Europäisches Patentamt

European Patent Office

Office européen des brevets

(11) Publication number: **0 094 039**
**A1**

(12) # EUROPEAN PATENT APPLICATION

(21) Application number: **83104428.4**

(22) Date of filing: **05.05.83**

(51) Int. Cl.³: **A 61 B 17/18**

(30) Priority: **12.05.82 IT 342082**

(43) Date of publication of application: **16.11.83**
**Bulletin 83/46**

(84) Designated Contracting States: **AT BE CH DE FR GB LI LU NL SE**

(71) Applicant: **Scaglietti, Oscar, Via Malvezza 2/2, I-40139 Bologna (IT)**

(72) Inventor: **Scaglietti, Oscar, Via Malvezza 2/2, I-40139 Bologna (IT)**

(74) Representative: **Modiano, Guido et al, MODIANO, JOSIF, PISANTY & STAUB Modiano & Associati Via Meravigli, 16, I-20123 Milan (IT)**

(54) **Endomedullar nail for long bones.**

(57) The invention relates to an endomedullar nail for long bones. The nail (1) comprises a stem in the form of a series of successive segments (3a, 3b) lying obliquely with respect to each other. Such segments (3a, 3b) define imaginary triangles (2a, 2b, 2c) having their bases on the stem axis (A) and their sides on a pair of said consecutive segments (3a, 3b). Such triangles (2a, 2c) are contained in axial planes evenly set at 120° angles from each other.

"ENDOMEDULLAR NAIL FOR LONG BONES"

This invention relates to an endomedullar nail for long bones.

It is known in the orthopedic art that to reduce long bone fractures, such as a fractured thighbone, nails are commonly used which are inserted lenghtwise through the     medullar duct.

Presently known are endomedullar nails of the so-called Ender type, which have a slightly bent, flattened cross-section stem. However, one of such nails cannot provide an adequately firm grip within the medullar duct.  It thus becomes necessary to employ a number of such nails arranged to lie on suitably offset longitudinal planes.

Also known are nails of the so-called Kuntscher type, having a tubular cross-sectional configuration, which tend to have a larger size and provide a more reliable grip, but the insertion whereof is more traumatic.

It is an object of this invention to obviate the deficiencies exhibited by conventional endomedullar nails, by providing an endomedullar nail which can give assurance of a firm grip, while being extremely easy to insert.

A further object of the invention is to provide an endomedullar nail of simple design, versatile in use, as well as of a relatively low cost.

The objects of the invention are achieved by an endomedullar nail for long bones, comprising a stem defining a mean geometrical reference axis, characterized in that said stem has the pattern of substantially polygonal line, at least some of the vertices of the angles of the polygonal line lying on points at a distance from said reference axis.

According to one embodiment the said points lie on an imaginary geometric surface of substantially cylindrical shape.

The invention features will be more readily apparent from the following detailed description of a preferred embodiment of this endomedullar nail for long bones, with reference to the accompanying illustrative drawing, where:

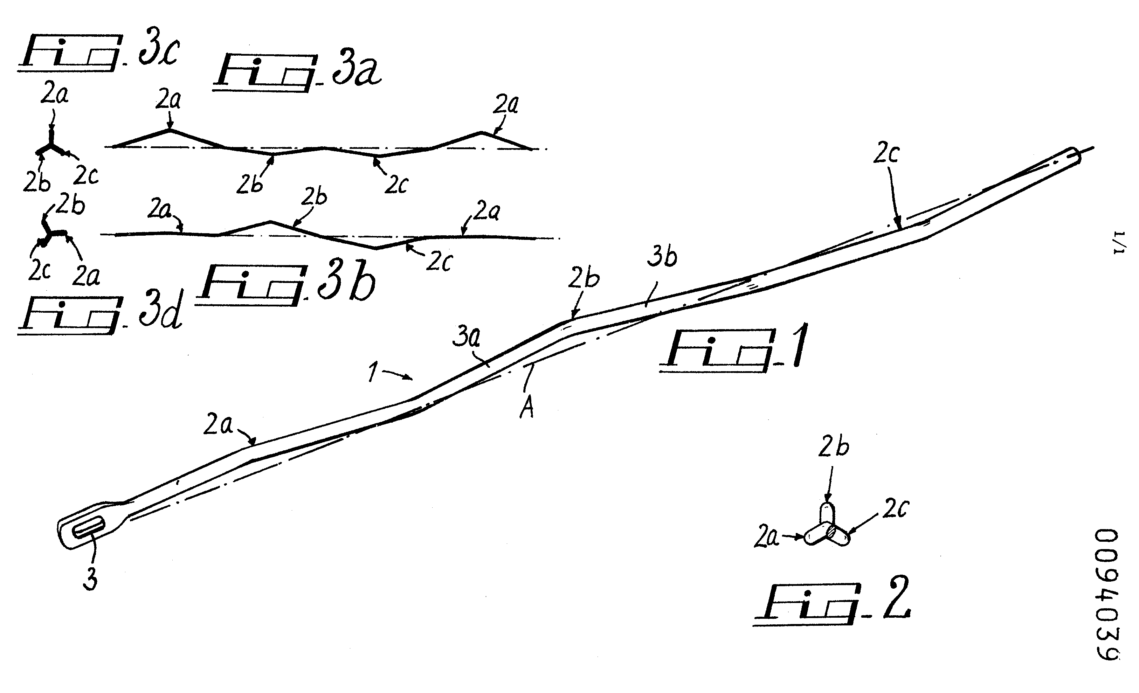

Figure 1 is a perspective view of this endomedullar nail;

Figure 2 is a cross-sectional view of the endomedullar nail;

Figures 3a, 3b show schematically two side elevation views of the nail taken respectively at 90° from each other; and

Figures 3c, 3d are side views of Figures 3a and 3b, respectively.

With reference to the drawing figures, generally indicated at 1 is this endomedullar nail for long bones. The nail 1 comprises essentially a metal stem of circular cross-sectional configuration and formed preferably from stainless steel. '

Said stem follows a broken or polygonal line pattern including a series of successive segments set slightly obliquely with respect to each other, so as to define a kind of imaginary triangles having their geometric bases on the mean axis A of the stem and two consecutive ones of such segments as their sides. Said triangles are contained in axial planes orderly set at angles of 120 degrees with respect to each other. Indicated at 2a, 2b, 2c, respectively, are in the drawing the geometric triangles which are formed by the stem on said angularly set planes, and at 3a, 3b,    the two segments which materially define each triangle.

In a conventional manner, the stem is also provided at one end with a slot 3 as an aid to insertion through the medullar  duct.

By virtue of its distinctive shape, this endomedullar nail can alone provide a reliable grip inside the medullar  duct. The vertices of the triangles 2a, 2b, 2c provide, in fact, as many bearing points lying, in an orderly offset arrangement, on one cylindrical surface whose axis is coincident with the stem axis.

Notwithstanding this, the nail can be easily and rapidly inserted, and adapt itself to fit the configuration of the medullar  duct.

It is, of course, possible to arrange for the triangles defined by the contiguous nail segments to extend on planes which may be set at different angles from each other.

It is also possible to arrange for the planes containing said triangles to form a triangular cross-section prism, thereby the nail stem would follow a broken line pattern which extends helically around the axis of the nail. In other words the nail of this modified embodiment has the shape of a coil or helix, in which the turns, instead of being of arcuated shape are of polygonal shape.

It has been found that in use it is advantageous to insert at least one or more of the nails of this invention in the medullar duct. If more nails are inserted, they are arranged therein in side by side relationship with the vertices offset with respect to each other.

In practicing the invention, the materials and dimensions may be any selected ones to meet individual requirements.

CLAIMS

1. An endomedullar nail for long bones, comprising a stem defining a mean geometrical reference axis, characterized in that said stem has the pattern of substantially polygonal line, at least some of the vertices of the angles of the polygonal line lying on points at a distance from said reference axis.

2. A nail according to Claim 1, characterized in that said points lie on an imaginary geometric surface of substantially cylindrical shape.

3. A nail according to Claim 1, characterized in that said stem has the form of a series of successive segments (3a, 3b) set obliquely with respect to each other so as to define a kind of geometric triangles (2a, 2b, 2c) having the bases thereof on the axis (A) of said stem and the sides on a pair of said consecutive segments, said triangles being arranged on axial planes orderly set at angles from each other.

4. A nail according to Claim 3, characterized in that said axial planes containing said consecutive segments pairs (3a, 3b) set at angles from each other are orderly set at angles of 120° from each other.

5. A nail according to Claim 1, characterized in that said stem has a circular cross-sectional configuration.

6. A nail according to Claim 3, characterized in that the nail has a generally helicoidal shape wherein the turns of the helix have a polygonal pattern.

7. A nail according to Claim 6, characterized in that the turns of the helix have a triangular pattern.

8. Use of the nail according to claims 1 to 6, characterized in that at least one of said nails is inserted in the medullar duct of the bone.

Fig. 3c

Fig. 3a

Fig. 3b

Fig. 3d

Fig. 1

Fig. 2

2a
2b 2c

2b
2c 2a

2a
2b 2c

2a
2b 2c

2a
2b

1

3a

2b

3b

A

2c

3

2a

2b
2a 2c

1/1

0094039

## EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | DE-A-1 930 354  (HALLORAN) | | A 61 B  17/18 |
| | --- | | |
| A | US-A-4 169 470  (ENDER) | | |
| | ----- | | |

TECHNICAL FIELDS SEARCHED (Int. Cl. ³)

A 61 B

The present search report has been drawn up for all claims

| Place of search | Date of completion of the search | Examiner |
|---|---|---|
| THE HAGUE | 22-08-1983 | LOWE D. |